# EUROPEAN PATENT SPECIFICATION

(11) **EP 2 593 533 B1**
(45) Date of publication and mention of the grant of the patent: **06.07.2016**
(21) Application number: 11807420.2
(22) Date of filing: 13.07.2011
(51) Int. Cl.: C10G 29/20, C07C 7/20, C07C 7/152

(54) **USE OF ALPHA-AMINO ETHERS FOR THE REMOVAL OF MERCAPTANS FROM HYDROCARBONS**
VERWENDUNG VON ALPHA-AMINOETHERN ZUR ENTFERNUNG VON MERCAPTANEN AUS KOHLENWASSERSTOFFEN
UTILISATION D'ALPHA-AMINO ÉTHERS POUR ÉLIMINER DES MERCAPTANS CONTENUS DANS DES HYDROCARBURES

(30) Priority: 14.07.2010 US 835881
(43) Date of publication of application: 22.05.2013
(73) Proprietor: Nalco Company, Naperville, IL 60563-1198 (US)
(72) Inventor: COMPTON, Dennis R., Sugar Land Texas 77478 (US)
(74) Representative: Godemeyer Blum Lenze Patentanwälte Partnerschaft mbB - werkpatent
(86) International application number: PCT/US2011/043778
(87) International publication number: WO 2012/009390

(56) References cited:
- EP-A2- 0 882 778
- WO-A1-2010/027353
- WO-A2-97/25126
- GB-A- 2 290 542
- US-A- 2 186 398
- US-A- 3 408 287
- US-A- 4 556 546
- US-A- 4 556 546
- US-A- 5 589 149
- US-A1- 2012 329 930

## Description

### Background of the Invention

The present invention relates to the scavenging of mercaptans in hydrocarbon fluids and more particularly to the use of alpha-amino ethers as mercaptan scavengers. Hydrocarbon fluids, such as crude oil, crude oil emulsions, oilfield condensate, petroleum residua and refined fuels such as naphtha, kerosene, gasoline, and other purified petroleum products often contain a variety of mercaptans, including mercaptans of relatively low molecular weight. Because of the volatility of these low molecular weight mercaptans (for example, methyl mercaptan, CH₃SH, ethyl mercaptan, CH₃CH₂SH, and propyl mercaptan, CH₃CH₂CH₂SH), they tend to evolve into vapor spaces, where their offensive odors create problems in and around storage areas and throughout pipelines and shipping systems used for transporting the hydrocarbon.

Various additives have been employed in efforts to alleviate these problems. For example, choline or choline hydroxide has been found to alleviate hydrogen sulfide evolution and to scavenge mercaptans as show in US Patents 4,594,147, 4,867,865, and 5,183,560. However, choline and choline hydroxide are not well suited for many uses and media, such as in crude oil. Although choline and choline hydroxide might scavenge mercaptans in such media, they also form a volatile and malodorous by-product with the sulfur compounds indigenous to such media. Accordingly, the use of choline and choline hydroxide to control odors associated with light weight mercaptans is self-defeating in media such as crude oil. European Patent Application 0 538 819 A3 describes the use of oil-soluble quaternary ammonium compounds to scavenge mercaptans from certain oils, especially high boiling, heavy residual fuels. US Patent 5,840,177 describes using quaternary ammonium hydroxides to scavenge mercaptans from certain hydrocarbon fluids.

European Patent Application 0 882 778 A2 describes a composition and method for sweeting gaseous or liquid hydrocarbons, aqueous systems and mixtures thereof with a non-regenerative scavenger of a specific chemical formula.

Despite these attempts however there is still a need for compositions that are produced in high yields and low cost, and that reduce mercaptan concentrations more effectively than the prior art. Thus there is clear need and utility for compositions and methods for scavenging molecular weight mercaptans more effectively and more efficiently. The art described in this section is not intended to constitute an admission that any patent, publication or other information referred to herein is "prior art" with respect to this invention, unless specifically designated as such.

### Brief Summary of the Invention

The invention is directed towards a method of reducing the amount of mercaptans in a hydrocarbon fluid according to claim 1.

The hydrocarbon fluid can be a liquid. The hydrocarbon fluid can be selected from the list consisting of but not limited to diesel fuel, kerosene, and gasoline.

The mercaptans can be present in the hydrocarbon fluid in amounts in excess of 500 ppm. The ratio of alpha-amino ether molecules to mercaptan molecules can be within the range of 1:1000 to 1000:1.

### Description of the Drawings

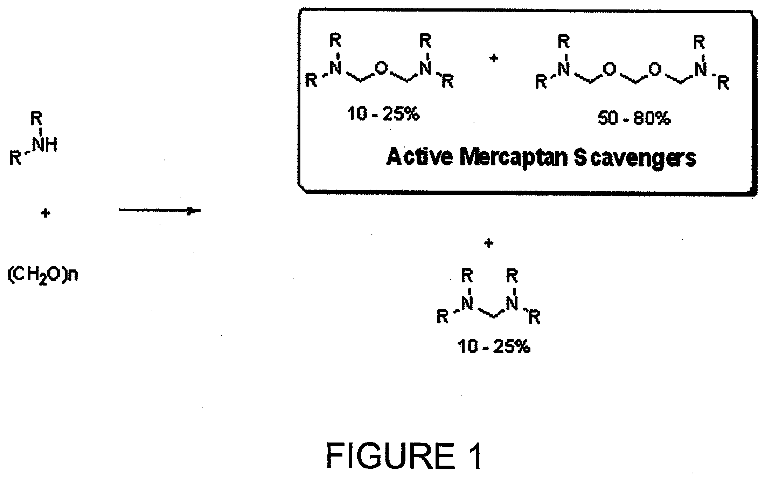

FIG.1 illustrates compositions of matter useful as a mercaptan scavenger.

### Detailed Description of the Invention

For purposes of this application the definition of these terms is as follows:
"Alpha-amino ether" means a molecule according to the formula: 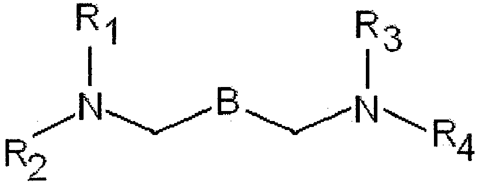 Where: R₁, R₂, R₃, R₄, are carbon containing side chains containing 1 - 20 carbon atoms and includes cyclic and acyclic compounds. The cyclic compounds can be aromatic or non-aromatic. Examples include but are not limited to, methyl, ethyl, propyl, tert-butyl, cyclopentyl, cyclohexyl, morpholino, and phenyl, and they all can be the same group or one or more different groups. B is an ether group, which is either an oxygen atom or a group having an oxygen atom at both ends (such as -OCH₂O- or -OC₂H₄O-).

"Hydrocarbon fluid" means a liquid or gas predominantly comprising organic material including but not limited to kerosene, crude oil, crude oil emulsions, oilfield condensate, petroleum residua, refined fuels, distillate fuels, fuel oil, heating oils, diesel fuel, gasoline, jet fuel, bunker fuel oils, and any combination thereof.

"Mercaptan" means a sulfur-containing organic compound with the general formula RSH where R is any alkyl, aromatic or cyclic group. Examples of common mercaptans are methylmercaptan CH₃SH, ethylmercaptan CH₃CH₂SH, propylmercaptan CH₃(CH₂)₂SH,

isopropylmercaptan (CH3)2CHSH, Phenylmercaptan C₅H₅SH.

"Scavenger" means a composition of matter, such as but not limited to alpha-amino ethers, useful in reducing the amount of or mitigating the effects of some other composition of matter, such as but not limited to mercaptans, in a fluid medium.

"Formaldehyde equivalent" means a composition of matter containing at least one group according to the formula: (CH₂O)ₙ in which n is an integer greater than or equal to 1, and/or a composition of matter including formaldehyde or related molecules such as paraformaldehyde, and/or s-trioxane.

In the event that the above definitions or a description stated elsewhere in this application is inconsistent with a meaning (explicit or implicit) which is commonly used, in a dictionary, or stated in a source incorporated by reference into this application, the application and the claim terms in particular are understood to be construed according to the definition or description in this application, and not according to the common definition, dictionary definition, or the definition that was incorporated by reference. In light of the above, in the event that a term can only be understood if it is construed by a dictionary, if the term is defined by the Kirk-Othmer Encyclopedia of Chemical Technology, 5th Edition, (2005), (Published by Wiley, John & Sons, Inc.) this definition shall control how the term is to be defined in the claims.

In the invention the amount of mercaptans in a hydrocarbon fluid is reduced by the introduction of an alpha-amino ether into the fluid. The alpha-amino ether scavenges mercaptan by forming stable non-volatile compounds The new sulfur containing compounds are higher molecular weight and lower volatility mitigating the odor associated with volatile mercaptans.

As illustrated in FIG. 1, in at least one embodiment the mercaptan scavenger is produced by reacting a secondary amine:with formaldehyde or other formaldehyde equivalents such as paraformaldehyde or s-trioxane. This produces a composition of matter that predominantly comprises two forms of alpha-amino ethers. 10%-25% of the composition is a single oxygen ether and 50%-80% of the composition is a double oxygen ether with a single carbon atom between the ether oxygen atoms. The reaction product also comprises 10-25% double amine that is non-reactive to the mercaptans. In FIG 1, any of the R groups correspond to any of the R₁, R₂, R₃, and R₄, groups described in the definition of "alpha-amino ether".

In at least one embodiment the reaction is performed without solvent. This reaction can be performed with solvents such as aromatic 150, aromatic 100, etc. to create a diluted product.

A scavenging formulation is used in a hydrocarbon fluid. The formulation can but is not required to contain a solvent and at least two alpha-amino ethers, the alpha-amino ethers can be used without solvent. The solvent is selected from the list consisting of water, alcohol, aromatic solvent, non-aromatic solvent and a solvent that solvates alpha-amino ethers, and any combination thereof. The formulation can be introduced into the hydrocarbon fluid by mechanical means including but not limited to injection pumps or any other mechanism known in the art including but not limited to those methods described in US Patent 5,840,177.

### EXAMPLES

The foregoing may be better understood by reference to the following example.

Samples of kerosene were treated with known amounts of mercaptans and were tested according to ASTM D5623-94. In the test procedure described by ASTM D5623-94 the oven temperature increases from 10 °C (50 °F) to 250 °C (482 °F) at a rate of 10 °C/min. Any sulfur containing compound that passes through the GC column up to

the endpoint and does not have a retention time calibrated to a known structure is listed as an unidentified volatile compound.

Table 1 shows the ppm wt of sulfur in the form of sec-butyl mercaptan and n-butyl mercaptan in the untreated sample. Tables 2 and 3 show the reduction in mercaptans when treated with alpha-amino ethers derived from butyl amine and morpholine respectively. The loss of n-butyl mercaptan and increase in unidentified volatile sulfur compounds indicates that the mercaptan has formed a stable compound that did not match a known standard.

### Untreated Sample

### Sulfur Speciation ASTM D-5623

**Table 1**

| *Mercaptan* | *ppm wt Sulfur* |
|---|---|
| sec-Butyl Mercaptan | 1.5 |
| n-Butyl Mercaptan | 396 |

### N,N'-oxybis(methylene)bis(N,N-dibutylamine) and N,N'-(methylenebis(oxy)bis(methylene))bis(N,N-dibutylamine) Sulfur Speciation ASTM D-5623

**Table 2**

| *Mercaptan* | *ppm wt Sulfur* | *Sulfur Reduction* | *% Reduction* |
|---|---|---|---|
| sec-Butyl Mercaptan | <0.2 | >1.3 | >87 |
| n-Butyl Mercaptan | 6.8 | 389.2 | 98.3 |
| Unidentified Volatile Sulfur | 388 | | |

### 4,4'-oxybis(methylene)dimorpholine and bis(morpholinomethoxy)methane Sulfur Speciation ASTM D-5623

**Table 3**

| *Mercaptan* | *ppm wt Sulfur* | *Sulfur Reduction* | *% Reduction* |
|---|---|---|---|
| sec-Butyl Mercaptan | 0.4 | 1.1 | 73 |
| n-Butyl Mercaptan | 45 | 351 | 88.6 |
| Unidentified Volatile Sulfur | 343 | | |

To further illustrate the effectiveness of alpha-amino ethers as mercaptan scavengers, 9 different mercaptans were mixed together (Tables 4 and 5). The mercaptan mixture (1000 ppm v/v) was added to kerosene and then the ppm wt of the sulfur species was measured according to ASTM D5623-94. Various alpha-amino ethers (Tables 6 - 10) were used to treat the mercaptans by adding 3000 ppm v/v of various alpha-amino ethers. The decrease in the individual mercaptan components and the corresponding increase in unidentified volatile sulfur species demonstrates that the mercaptans reacted with the alpha-amino ethers to from stable compounds of lower volatility.

### A complex mercaptan solution of nine different mercaptans was prepared and a series of alpha amino ethers was tested for their ability to reduce the mercaptans. 1000 ppm (v/v) was added to a solution of kerosene. The kerosene was then treated with 3000 ppm (v/v) of the specified samples.

**Tables 4 and 5**

| | | | | **Untreated Sample Sulfur Speciation ASTM D-5623** | |
|---|---|---|---|---|---|
| *Mercaptan* | *Mass* % | *Mole %* | | | *ppm wt Sulfur* |
| Ethyl | 11.1 | 15.7 | | *Mercaptan* | |
| Isopropyl | 11.1 | 12.8 | | Ethyl | 57 |
| tert-Butyl | 11.1 | 10.8 | | Isopropyl | 48 |
| n-Propyl | 11.1 | 12.8 | | tert-Butyl | 45 |
| Isobutyl | 11.1 | 10.8 | | N-Propyl | 49 |
| n-Butyl | 11.1 | 10.8 | | Isobutyl | 42 |
| Phenyl | 11.1 | 8.9 | | N-Butyl | 43 |
| Benzyl | 11.1 | 7.9 | | Phenyl | 35 |
| n-Amyl | 11.1 | 9.5 | | Benzyl | 33 |
| | | | | Unidentified (n-Amyl) | 40 |

### N,N'-oxybis(methylene)bis(N,N-dibutylamine)and N,N'-(methylenebis(oxy)bis(methylene))bis(N,N-dibutylamine) Sulfur Speciation ASTM D-5623

**Table 6**

| *Mercaptan* | *ppm wt Sulfur* | *Sulfur Reduction* | *% Reduction* |
|---|---|---|---|
| Ethyl | 0.4 | 56.6 | 99.3 |
| Isopropyl | 0.4 | 47.6 | 99.2 |
| tert-Butyl | 1.0 | 44.0 | 97.8 |
| N-Propyl | 0.3 | 48.7 | 99.4 |
| Isobutyl | 0.4 | 41.6 | 99.0 |
| N-Butyl | 0.4 | 42.6 | 99.1 |
| Phenyl | 0.8 | 34.2 | 97.7 |
| Benzyl | 0.6 | 32.4 | 98.2 |
| Unidentified | 416 | | |

### 1,1'-oxybis(methylene)dipiperidine and bis(piperidinomethoxy)methane Sulfur Speciation ASTM D-5623

**Table 7**

| *Mercaptan* | *ppm wt Sulfur* | *Sulfur Reduction* | % *Reduction* |
|---|---|---|---|
| Ethyl | 0.2 | 56.8 | 99.6 |
| Isopropyl | <0.2 | >47.8 | >99.6 |
| tert-Butyl | 0.3 | 44.7 | 99.3 |
| N-Propyl | 0.2 | 48.8 | 99.6 |
| Isobutyl | <0.2 | >41.8 | >99.6 |
| N-Butyl | <0.2 | >42.8 | >99.6 |
| Phenyl | 0.3 | 34.7 | 99.1 |
| Benzyl | 0.4 | 32.6 | 98.8 |
| Unidentified | 431 | | |

### 4,4'-oxybis(methylene)dimorpholine and bis(morpholinomethoxy)methane Sulfur Speciation ASTM D-5623

**Table 8**

| *Mercaptan* | *ppm wt Sulfur* | *Sulfur Reduction* | % *Reduction* |
|---|---|---|---|
| Ethyl | <0.2 | >56.8 | >99.6 |
| Isopropyl | <0.2 | >47.8 | >99.6 |
| tert-Butyl | 0.2 | 44.8 | 99.6 |
| N-Propyl | <0.2 | >48.8 | >99.6 |
| Isobutyl | 0.3 | 41.7 | 99.3 |
| N-Butyl | 0.2 | 42.8 | 99.5 |
| Phenyl | 0.4 | 34.6 | 98.9 |
| Benzyl | 1.5 | 31.5 | 95.5 |
| Unidentified | 437 | | |

### 1,1'-oxybis(methylene)dipyrrolidine and bis(pyrrolidinomethoxy)methane Sulfur Speciation ASTM D-5623

**Table 9**

| *Mercaptan* | *ppm wt Sulfur* | *Sulfur Reduction* | % *Reduction* |
|---|---|---|---|
| Ethyl | 0.2 | 56.8 | 99.6 |
| Isopropyl | <0.2 | >47.8 | >99.6 |
| tert-Butyl | 0.2 | 44.8 | 99.6 |
| N-Propyl | 0.2 | 48.8 | 99.6 |
| Isobutyl | 0.2 | 41.8 | 99.5 |
| N-Butyl | 0.2 | 42.8 | 99.5 |
| Phenyl | 0.4 | 34.6 | 98.9 |
| Benzyl | <0.2 | >32.8 | >99.4 |
| Unidentified | 411 | | |

### N,N'-oxybis(methylene)bis(N,N-diethylamine) and N,N'-(methylenebis(oxy)bis(methylene))bis(N,N-diethylamine) Sulfur Speciation ASTM D-5623

**Table 10**

| *Mercaptan* | *ppm wt Sulfur* | *Sulfur Reduction* | % *Reduction* |
|---|---|---|---|
| Ethyl | 0.2 | 56.8 | 99.6 |
| Isopropyl | 0.2 | 47.8 | 99.6 |
| tert-Butyl | 0.3 | 44.7 | 99.3 |
| N-Propyl | 0.2 | 48.8 | 99.6 |
| Isobutyl | <0.2 | >41.8 | >99.5 |
| N-Butyl | 0.2 | 42.8 | 99.5 |
| Phenyl | <0.2 | >34.8 | >99.4 |
| Benzyl | <0.2 | >32.8 | >99.4 |
| Unidentified | 436 | | |

The previous data makes clear that the addition of an alpha-amino ether to a hydrocarbon fluid reduces the mercaptan to a non-volatile sulfur species and thereby scavenges mercaptans from the hydrocarbon fluid.

## Claims

1. A method of reducing the amount of mercaptans in a hydrocarbon fluid comprising introducing an alpha-amino ether into the fluid, the alpha-amino ether scavenges mercaptan by forming stable non-volatile compounds, the new sulfur containing compounds are higher molecular weight and lower volatility mitigating the odor associated with volatile mercaptans, wherein the alpha-amino ether is selected from the list consisting of:
a combination of 1,1'-oxybis(methylene)dipiperidine and bis(piperidinomethoxy)methane, and
a combination of 1,1'-oxybis(methylene)dipyrrolidine and bis(pyrrolldinomethoxy)methane.

2. The method of claim 1 wherein the hydrocarbon fluid is liquid.

3. The method of claim 1 wherein the hydrocarbon fluid is selected from the list consisting of products produced from crude oils such as but not limited to kerosene, diesel fuel, gasoline, naphtha and heavy aromatic naphtha.

4. The method of claim 1 wherein there are different kinds of mercaptans in the hydrocarbon fluid.

## Patentansprüche

1. Verfahren zur Verringerung der Menge an Mercaptanen in einem Kohlenwasserstofffluid, umfassend das Einführen eines alpha-Aminoethers in das Fluid, wobei der alpha-Aminoether Mercaptan abfängt durch Bildung stabiler, nicht-flüchtiger Verbindungen, wobei die neuen schwefelhaltigen Verbindungen ein höheres Molekulargewicht und eine geringere Flüchtigkeit aufweisen, was den mit flüchtigen Mercaptanen verbundenen Geruch abschwächt, wobei der alpha-Aminoether ausgewählt ist aus der Liste, welche besteht aus:
einer Kombination von 1,1'-Oxybis(methylen)dipiperidin und Bis(piperidinomethoxy)methan, sowie
einer Kombination von 1,1'-Oxybis(methylen)dipyrrolidin und Bis(pyrrolidinomethoxy)methan.

2. Verfahren nach Anspruch 1, wobei das Kohlenwasserstofffluid flüssig ist.

3. Verfahren nach Anspruch 1, wobei das Kohlenwasserstofffluid ausgewählt ist aus der Liste, welche besteht aus Produkten, hergestellt aus Rohölen, wie beispielsweise, jedoch nicht darauf beschränkt, Kerosin, Dieselkraftstoff, Benzin, Naphtha und schwerem aromatischem Naphtha.

4. Verfahren nach Anspruch 1, wobei verschiedene Arten von Mercaptanen im Kohlenwasserstofffluid vorliegen.

## Revendications

1. Procédé de réduction de la quantité de mercaptans dans un fluide hydrocarbure, comprenant l'introduction d'un alpha-amino éther dans le fluide, l'alpha-amino éther piégeant le mercaptan en formant des composés non-volatils stables, les nouveaux composés contenant du soufre ayant une masse moléculaire supérieure et une volatilité inférieure atténuant l'odeur associée aux mercaptans volatils, dans lequel l'alpha-amino éther est sélectionné dans la liste constituée de :
une combinaison de 1,1'-oxybis(méthylène)dipipéridine et de bis(pipéridinométhoxy)méthane, et
une combinaison de 1,1'-oxybis(méthylène)dipyrrolidine et de bis(pyrrolidinométhoxy)méthane.

2. Procédé selon la revendication 1, dans lequel le fluide hydrocarbure est un liquide.

3. Procédé selon la revendication 1, dans lequel le fluide hydrocarbure est sélectionné dans la liste constituée de produits réalisés à partir de pétroles bruts, tels que, sans y être limités, du kérosène, du combustible diesel, de l'essence, du naphta et du naphta aromatique lourd.

4. Procédé selon la revendication 1, dans lequel il existe différents types de mercaptans dans le fluide hydrocarbure.
